Europäisches Patentamt

European Patent Office

Office européen des brevets

(11) Veröffentlichungsnummer: **0 466 956 A1**

## (12) EUROPÄISCHE PATENTANMELDUNG

(21) Anmeldenummer: **90113797.6**

(22) Anmeldetag: **18.07.90**

(43) Veröffentlichungstag der Anmeldung:
**22.01.92 Patentblatt 92/04**

(84) Benannte Vertragsstaaten:
**DE FR GB**

(51) Int. Cl.⁵: **A61B 6/03**

(71) Anmelder: **SIEMENS AKTIENGESELLSCHAFT**
**Wittelsbacherplatz 2**
**W-8000 München 2(DE)**

(72) Erfinder: **Schittenhelm, Rudolf, Dr. rer. nat.**
**von Bezzelstrasse 6**
**W-8520 Erlangen(DE)**

(54) **Computertomograph.**

㊼ Bei einem Computertomograph mit einer ringförmigen Anode (6), einem von dieser umschlossenen, koaxial dazu liegenden Detektorring (15) und einem ebenfalls koaxial zur Anode (6) liegenden Kathodenring mit einer Reihe von Kathoden (7) wird deren Elektronenstrahlung zur Anode (6) zur Fokusverschiebung schrittweise freigegeben.

Jeder Kathode (7) ist eine Ablenkvorrichtung (40) zum Ablenken des erzeugten Fokus(F) in Umfangsrichtung der Anode (6) zugeordnet. Eine oder mehrere Kathode(n) (7) kann bzw. können mit der zugeordneten Ablenkvorrichtung (40) und einem Anodenstück als Modul ausgebildet sein.

Bei einem solchen Computertomograph wird die Anzahl der Fokuspositionen gesteigert und die Zeit zur Erfassung eines Bilddatensatzes wesentlich reduziert.

FIG 3

EP 0 466 956 A1

Die Erfindung betrifft einen Computertomograph mit einer ringförmigen Anode, einem von dieser umschlossenen, koaxial dazu liegenden Detektorring und einem ebenfalls koaxial zur Anode liegenden Kathodenring mit einer Reihe von Kathoden, deren Elektronenstrahlung zur Anode zur schrittweisen Fokusverschiebung schrittweise freigegeben wird.

Bei einem Computertomograph dieser Art steht während der Abtastung des Untersuchungsobjektes unter verschiedenen Winkeln durch ein fächerförmiges Röntgenstrahlenbündel sowohl die Anode als auch der Detektorring und der Kathodenring fest. Es ist demgemäß eine sehr schnelle Abtastung des Untersuchungsobjektes um einen Gesamtwinkel von 360° möglich. Die Bildaufnahmezeit kann demgemäß sehr kurz sein.

Da die einzelnen Kathodensysteme des Kathodenringes eine endliche Breite haben, ist die Zahl der im Kathodenring einsetzbaren Kathodensysteme und damit die Zahl der möglichen Fokuspositionen begrenzt. Bei einem Mittenabstand der Kathodensysteme von beispielsweise 4 mm können auf der Anode mit einem üblichen Umfang von etwa 4 m nur etwa 1000 Fokuspositionen untergebracht werden. Der Mittenabstand der Kathodensysteme ist zur Erhöhung der Zahl der möglichen Fokuspositionen nicht beliebig verkleinerbar.

Der Erfindung liegt die Aufgabe zugrunde, einen Computertomograph der eingangs genannten Art so auszubilden, daß eine hohe Zahl von Fokuspositionen und damit eine hohe Bildqualität sichergestellt ist.

Diese Aufgabe ist erfindungsgemäß dadurch gelöst, daß jeder Kathode eine Ablenkvorrichtung zum Ablenken des erzeugten Fokus in Umfangsrichtung der Anode zugeordnet ist. Durch jedes Kathodensystem ist demgemäß nicht nur eine einzige Fokusposition, sondern eine Vielzahl von Fokuspositionen durch Ablenkung der Elektronenstrahlung möglich. Dadurch ergibt sich eine hohe Anzahl von insgesamt möglichen Fokuspositionen.

Zur Erfassung der jeweiligen Fokusposition kann zwischen der Anode und einer ein fächerförmiges Röntgenstrahlenbündel formenden, ringförmigen Schlitzblende ein zweiter Detektorring vorgesehen sein. Dieser zweite Detektorring kann aus Einzeldetektoren aufgebaut sein, wobei dann der jeweils ein Ausgangssignal liefernde Einzeldetektor die Fokusposition kennzeichnet. Es ist aber auch möglich, vor dem zweiten Detektorring einen Blendenring mit Öffnungen zum Durchtritt der Röntgenstrahlung vorzusehen, bei dem die Öffnungen nach Art eines Codes angeordnet und ausgebildet sind. Anhand der Signale des zweiten Detektorringes ist dann eine sehr genaue Bestimmung der jeweiligen Fokusposition möglich.

Die Erfindung ist nachfolgend anhand der Zeichnung näher erläutert. Es zeigen:

Fig. 1 einen Schnitt durch einen Computertomograph zur Erläuterung des Erfindungsgedankens,

Fig. 1a eine Einzelheit des Computertomograph gemäß Fig. 1,

Fig. 2 einen Schnitt durch den Computertomograph gemäß Fig. 1 nach der Linie II-II,

Fig. 3 bis 5 verschiedene Kathodensysteme des Computertomograph nach den Fig. 1, 1a und 2,

Fig. 6 Mittel zur Erfassung der Fokusposition bei dem Computertomograph gemäß den Fig. 1 und 2, und

Fig. 7 eine Einzelheit der Fig. 6.

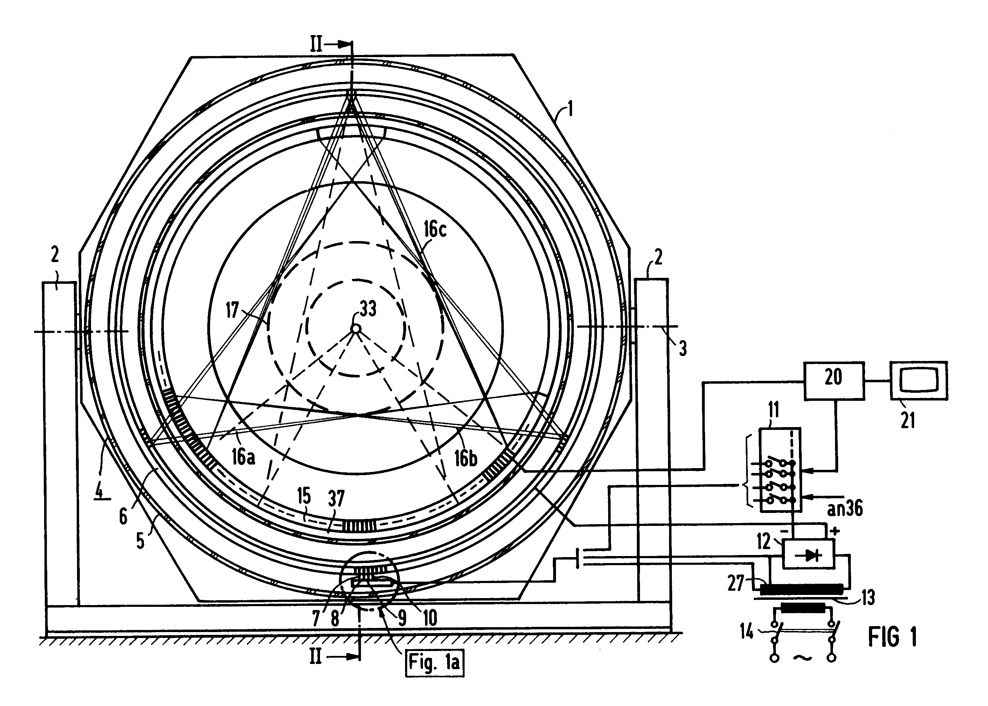

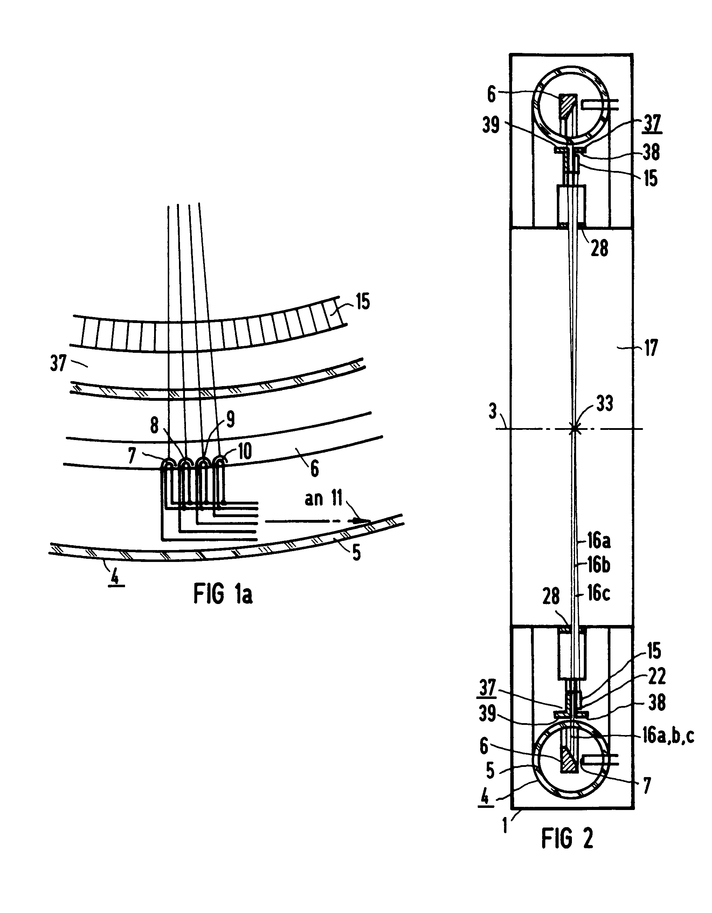

Der in den Fig. 1, 1a, 2 dargestellte Computertomograph weist ein Gehäuse 1 auf, das an zwei Stützen 2 um eine horizontale Achse 3 schwenkbar gelagert ist. Durch Schwenkung des Gehäuses 1 um die Achse 3 kann die Lage der gewünschten Transversalschicht des untersuchten Aufnahmeobjektes gewählt werden. Im Gehäuse 1 ist eine Röntgenstrahlenquelle 4 angeordnet, welche einen evakuierten Glasring 5 aufweist, in dem eine ebenfalls als Ring ausgebildete Anode 6 angeordnet ist. Der Anode 6 ist eine Vielzahl von Kathoden 7, 8, 9, 10 usw. zugeordnet. Die Kathoden 7 usw. sind an einer Schaltvorrichtung 11 angeschlossen, welche den negativen Pol eines Hochspannungsgleichrichters 12, der von einem Hochspannungstransformator 13 gespeist wird, schrittweise mit den Kathoden 7 usw. verbindet. Der positive Pol des Hochspannungsgleichrichters 12 ist mit der Anode 6 verbunden. Die Primärwicklung des Hochspannungstransformators 13 ist über einen Hauptschalter 14 ans Netz anschaltbar.

Damit eine schnelle Bildaufnahme innerhalb kürzester Zeit möglich ist, sind alle Kathoden 7 usw. gleichzeitig geheizt, d.h. gleichzeitig an einem Heiztransformator angeschlossen, der Teil des Hochspannungstransformators 13 ist und in der Zeichnung mit 27 bezeichnet ist. Zur Einschaltung der Röntgenstrahlung unter einem bestimmten Winkel ist also nur die Hochspannung an die entsprechende Kathode anzuschalten. Die Röntgenstrahlung tritt dann sofort auf.

Es ist auch möglich, zwischen jeder Kathode 7 usw. und der Anode 6 je ein Steuergitter anzuordnen und eine Schaltvorrichtung vorzusehen, durch die jedes Steuergitter zum aufeinanderfolgenden Einschalten der Elektronenstrahlung an eine diese freigebene Spannung anlegbar ist.

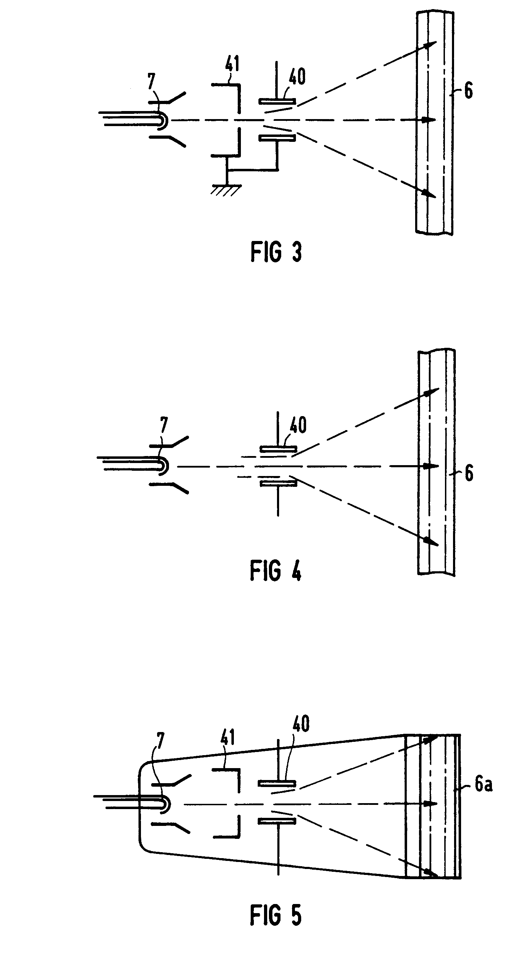

Zur kontinuierlichen Führung des Röntgenfokus über den Anodenring zeigt die Fig. 3 zwischen der Kathode 7 und der Anode 6 eine Vorrichtung 40 zum Ablenken der Elektronenstrahlung von der Kathode 7 zur Anode 6. Analog dazu arbeitende Ablenkvorrichtungen sind auch den anderen Kathoden zugeordnet. Dadurch ist erreicht, daß jeder Kathode nicht nur ein Fokus, sondern eine Fokusbahn zugeordnet ist, wodurch die Gesamtzahl der für die Bildrekonstruktion benutzten Foken und damit die Bildqualität steigt. Die Ablenkvorrichtung 40 besteht aus zwei Kondensatorplatten, die mit einer sägezahnförmigen Spannung beaufschlagt werden. Zwischen ihr und der Kathode 7 ist ein Gitter 41 zur Strahlformung angeordnet.

Auf das Gitter 41 kann unter Umständen auch verzichtet werden, wie dies die Fig. 4 zeigt.

Die Fig. 5 zeigt, daß die Kathode 7, die Ablenkvorrichtung 40 und ein Anodenstück 6a zu einem Modul zusammengefaßt sind. Eine Vielzahl solcher Module bildet einen Ring, der konzentrisch zum Mittelpunkt 33 der Röntgenstrahlenquelle 4 liegt. Solche Module können auch jeweils mehrere Kathoden enthalten.

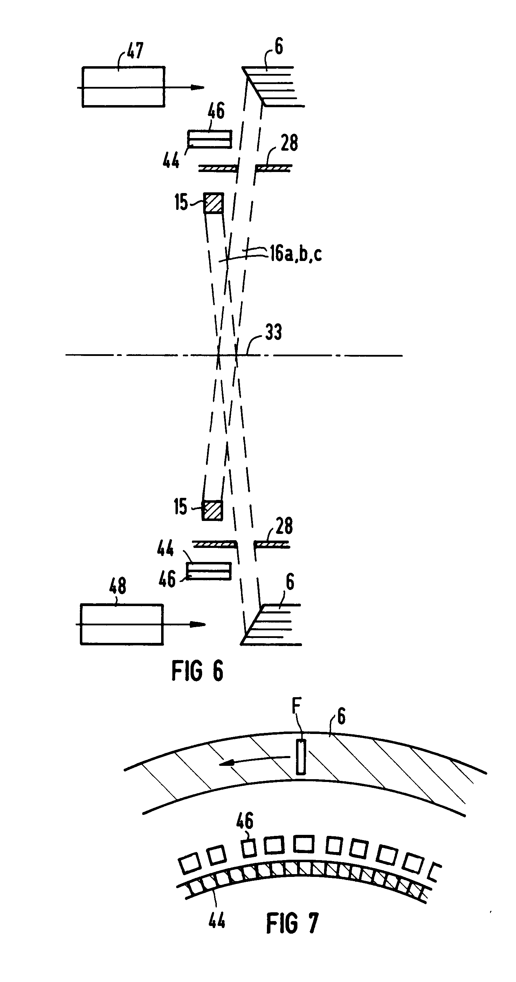

Zur Erfassung der jeweiligen Fokusposition ist außer einem einen Detektorring bildenden Strahlenempfänger 15 ein zweiter Detektorring 44 aus einer Anzahl von Einzeldetektoren vorgesehen, der zwischen der Anode 6 und einer die fächerförmigen Röntgenstrahlenbündel 16a, b, c formenden, ringförmigen Schlitzblende 28 angeordnet ist. In der Fig. 6 sind diese Komponenten sowie eine in Strahlrichtung vor dem zweiten Detektorring 44 angeordnete, ringförmige Blende 46 und zwei schematisch dargestellte Kathodensysteme 47, 48 gezeigt.

Die Fig. 6 zeigt, daß die von der Anode 6 ausgehende Röntgenstrahlung durch die Blende 46, und zwar durch darin vorgesehene Schlitze auf den Detektorelementen des Detektorringes 44 auftrifft. Aus deren Ausgangssignalen kann auf die jeweilige Fokusposition, die in der Fig. 7 beispielsweise mit F bezeichnet ist, geschlossen werden.

Eine genaue Erfassung der Fokusposition ist möglich, wenn die Schlitze der Blende 46 ungleichmäßig sind, da sich dann eine Codierung der Ausgangssignale der Detektorelemente des Detektorringes 44 ergibt (Fig. 7).

Die Röntgenstrahlenquelle 4 umschließt konzentrisch den ebenfalls als Kreisring ausgebildeten Strahlenempfänger 15, welcher aus einer Reihe einzelner Detektoren besteht, deren Anzahl von der gewünschten Meßwertzahl abhängt. Zur Einblendung der fächerförmigen Röntgenstrahlenbündel 16a, b, c ist die Blendenanordnung 28 vorgesehen.

Bei dem Ausführungsbeispiel nach Fig. 1 sind drei gegeneinander versetzte Röntgenstrahlenbündel 16a, b, c vorgesehen, so daß eine sehr schnelle Abtastung des Aufnahmeobjektes möglich ist. Ein Röntgenstrahlenbündel braucht nämlich nur einen Winkel von etwa 120° zu überstreichen. Im Rahmen der Erfindung ist es je nach Streustrahlenkorrektur auch möglich, nur zwei oder nur ein Röntgenstrahlenbündel zu nutzen. In diesem Fall muß ein Röntgenstrahlenbündel einen Winkel von 180° bzw. 360° überstreichen. Auch dabei ist eine sehr schnelle Abtastung wegen der elektronischen Fokusfortschaltung sichergestellt.

Die Schaltvorrichtung 11 ist so ausgebildet, daß mehrere, und zwar drei Röntgenstrahlenbündel 16a, 16b und 16c gleichzeitig aus verschiedenen Richtungen den Patienten durchsetzen und synchron zueinander in gleichen Schritten wandern. Die Zentralstrahlen der Röntgenstrahlenbündel 16a, b, c sind dabei um 120° gegeneinander versetzt.

Der Strahlenempfänger 15 ist seitlich an einer ringförmigen Spaltblende 37 angeordnet, welche senkrecht zur Schichtebene die Röntgenstrahlenbündel 16a, b, c begrenzt und Ansätze 38, 39 zur Abschirmung der auf das Aufnahmeobjekt gerichteten Röntgenstrahlung besitzt.

Das Gehäuse 1 besitzt eine zentrische Öffnung 17, in die eine Liege mit einem Patienten einführbar ist.

Der Strahlenempfänger 15 ist an einem Meßwertumformer 20 angeschlossen, welcher aus den Meßwerten ein Bild der durchstrahlten Transversalschicht des Patienten berechnet und seine Wiedergabe auf einem Sichtgerät 21 bewirkt.

Die Röntgenstrahlenbündel 16a, b, c und der Strahlenempfänger 15 sind so aufeinander ausgerichtet, daß die Röntgenstrahlenbündel in Strahlrichtung gesehen zunächst seitlich am Strahlenempfänger 15 an der Stelle 22 vorbei verlaufen und auf ihm auftreffen, wenn sie den Patienten durchstrahlt haben.

Zur Untersuchung eines Patienten, d.h. zur Aufnahme eines Schichtbildes, werden zunächst drei Schalter der Schaltvorrichtung 11 geschlossen. Dadurch wird Hochspannung zwischen die Anode und drei Kathoden gelegt und die Röntgenstrahlenbündel 16a, b, c z.B. in den in der Fig. 1 dargestellten Richtungen ausgestrahlt. Nachdem der Meßwertumformer 20 die Meßwerte des Strahlenempfängers 15 verarbeitet hat, gibt er an die Schaltvorrichtung 11 ein Signal zur Öffnung dieser Schalter und zum Schließen der nächsten Schalter. Die Symmetrieachse der Röntgenstrahlenbündel wandert daher um z.B. 0,1° im Uhrzeigersinn weiter. Nachdem der Meßwertumformer 20 wieder die Meßwerte des Strahlenempfängers 15 abgefragt hat, gibt er an die Schaltvorrichtung 11 ein Signal zum Öffnen dieser Schalter und zum Schließen der folgenden Schalter. Die Symmetrieachse der Röntgenstrahlenbündel wandert somit wieder um 0,1° weiter. Die schrittweise Verdrehung der Röntgenstrahlenbündel wiederholt sich so lange, bis jedes Röntgenstrahlenbündel einen vorbestimmten Winkelbereich von beispielsweise 120° überstrichen hat. Dann ist die Bildaufnahme beendet und das berechnete Querschnittsbild kann auf dem Sichtgerät 21 wiedergegeben werden.

Aus der Fig. 1 ergibt sich, daß die Röntgenstrahlenbündel 16a, b, c fächerförmig und in der Schichtebene so groß sind, daß der ganze Patient gleichzeitig durchstrahlt wird. Die Ausdehnung der Röntgenstrahlenbündel senkrecht zur Schichtebene ist etwa gleich der Schichtstärke.

**Patentansprüche**

1. Computertomograph mit einer ringförmigen Anode (6), einem von dieser umschlossenen, axial dazu liegenden Detektorring (15) und einem ebenfalls koaxial zur Anode liegenden Kathodenring mit einer Reihe von Kathoden (7 usw.), deren Elektronenstrahlung zur Anode (6) schrittweise freigegeben wird, **dadurch gekennzeichnet**, daß jeder Kathode (7 usw.) eine Ablenkvorrichtung (40) zur Verschiebung des erzeugten Fokus in Umfangsrichtung der Anode (6) zugeordnet ist.

2. Computertomograph nach Anspruch 1, **dadurch gekennzeichnet** daß eine oder mehrere Kathode(n) (7 usw.) mit der zugeordneten Ablenkvorrichtung (40) und einem Anodenstück (41) als Modul ausgebildet sind.

3. Computertomograph nach Anspruch 1 oder 2, **dadurch gekennzeichnet,** daß zwischen der Anode (6) und einer ein fächerförmiges Röntgenstrahlenbündel (16a, b, c) formenden, ringförmigen Schlitzblende (28) ein zweiter Detektorring (44) zur Erfassung der jeweiligen Fokusposition (F) angeordnet ist.

FIG 1a

FIG 2

**FIG 3**

**FIG 4**

**FIG 5**

FIG 6

FIG 7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | GB-A-2 034 149   (TOKYO SHIBAURA DENKI K.K.) <br> * Seite 1, Zeilen 70-88; Seite 2, Zeilen 16-47; Seite 4, Zeilen 1-59; Seite 5, Zeilen 34-76; Abblildungen 1-3,7,15-19 * <br> – – – | 1-3 | A 61 B 6/03 |
| Y | FR-A-2 368 930   (SIEMENS AG) <br> * Seite 3, Zeile 34 - Seite 6, Zeile 13 * <br> – – – | 1,3 | |
| Y | DE-A-2 812 644   (HIGH VOLTAGE ENGINEERING CORP.) <br> * Seite 9, Zeile 25 - Seite 10, Zeile 10; Seite 14, Zeile 24 - Seite 16, Zeile 25; Seite 17, Zeile 15 - Seite 18, Zeile 5; Seite 24, Zeile 18 - Seite 26, Zeile 15; Abbildungen 1,4-9 * <br> – – – | 1,3 | |
| A | DE-A-2 708 612   (NIHON DENSHI K.K.) <br> * Seite 6, Zeile 6 - Seite 7, Zeile 22; Seite 11, Zeile 9 - Seite 13, Zeile 29; Seite 15, Zeile 1 - Seite 16, Zeile 31; Abbildungen 1,7,8 * <br> – – – – – | 1-3 | |

| | RECHERCHIERTE SACHGEBIETE (Int. Cl.5) |
|---|---|
| | A 61 B |

Der vorliegende Recherchenbericht wurde für alle Patentansprüche erstellt

| Recherchenort | Abschlußdatum der Recherche | Prüfer |
|---|---|---|
| Den Haag | 13 März 91 | FONTENAY P.H.E.V. |